Europäisches Patentamt

European Patent Office

Office européen des brevets

(11) Publication number: **0 298 605 B1**

# EUROPEAN PATENT SPECIFICATION

(45) Date of publication of patent specification: **18.08.93**    (51) Int. Cl.5: **C12N 1/04, A21D 8/04**

(21) Application number: **88305205.2**

(22) Date of filing: **08.06.88**

(54) **Process for producing a stable bacterial composition and process for breadmaking using this composition.**

(30) Priority: **10.06.87 GB 8713601**

(43) Date of publication of application:
**11.01.89 Bulletin 89/02**

(45) Publication of the grant of the patent:
**18.08.93 Bulletin 93/33**

(84) Designated Contracting States:
**AT BE CH DE ES FR GB GR IT LI NL**

(56) References cited:
EP-A- 0 131 114
DE-A- 462 254
DE-A- 481 614
DE-A- 481 615
US-A- 2 938 794

S.E. GILLILAND: "Bacterial Starter Cultures
for Foods", 1985, pages 154-156, CRC Press,
Inc., Boca Raton, Florida, US

(73) Proprietor: **UNILEVER PLC**
**Unilever House Blackfriars P.O. Box 68**
**London EC4P 4BO(GB)**

(84) Designated Contracting States:
**GB**

(73) Proprietor: **UNILEVER N.V.**
**Weena 455**
**NL-3013 AL Rotterdam(NL)**

(84) Designated Contracting States:
**BE CH DE ES FR GR IT LI NL AT**

(72) Inventor: **Klapwijk, Pieter Marie**
**Lingedal 6**
**NL-2904 CK Capelle aan de Ijssel(NL)**
Inventor: **Klempp, Jurgen**
**Clemens-Hogg-Weg 18**
**D-7910 Neu-Ulm/Pfuhl(DE)**
Inventor: **van Rhee, Renee**
**Crabethstraat 33**
**NL-3141 XA Maassluis(NL)**

(74) Representative: **Dries, Antonius Johannes Ma-**
**ria et al**
**Unilever N.V. Patent Division P.O. Box 137**
**NL-3130 AC Vlaardingen (NL)**

EP 0 298 605 B1

## Description

This invention relates to a method for preparing supported microflora compositions suitable for fermentation processes used in the food, animal feed and agriculture industries, in particular for the preparation of bread by the sour-dough method. The invention results in improved supported Lactobacillus flora for use in such methods.

Fermentation plays an essential role in many food industries including the preparation of animal feedstuffs. Different micro-organisms are applied according to requirements. For example, yeast cells are applied in bakeries and breweries.

Before bread is baked from wheat flour, the dough from which it is prepared is usually aerated by the production of gas trapped in bubbles in the dough and generated by biological or chemical action. Usually baker's yeast is used for this leavening process. In baking the gas expands more while the continued heat kills further gas-forming action and the dough layers surrounding the gas bubbles harden with the effect that the baked bread substantially retains the shape of the risen dough. Yeast is a eukaryotic organism which due to the robust structure provided by the presence of a cellulose cell wall, may be readily provided in the form of instant dried yeast with a long shelf-life from which it is readily activated for use.

Whereas the introduction of yeast into the batter from which the dough is prepared, is a sufficient source of carbon dioxide by fermentation for leavening purposes, some types of bread require acid for taste and/or textural reasons. In baking rye bread or mixed wheat and rye bread especially, acid formation and pH reduction particularly below pH5 is required to suppress amylase activity in the dough and improve product taste. Yeast does not form acid however, and traditionally rye breads are baked according to the sour-dough method. In common with the preparation of many dairy and other food products therefore, this action is dependent on the development of a bacterial flora mainly consisting of a range of Lactic acid bacteria which provides the fermentative action. In many other examples in the food and animal feedstuffs industries the preservative, flavour and physical structure of the product is dependent on the application of active lactic acid bacteria.

Lactic acid bacteria are not at all related to yeast. They are prokaryotes, the cells are much smaller with different growth requirements and the cell wall structure is fundamentally different. One of the technological implications is that lactic acid bacteria in the same form as dried yeast or pressed yeast is so far unavailable and for example the concentration of lactic acid bacterial cells to high density for instance by centrifugation is very difficult.

As a result of these differences in the traditional sour-dough baking method a portion of old sour-dough from the previous batch of dough is incorporated into each successive dough batch. The use of successive generations of sour-dough in this fashion leads to unpredictable variations in the composition of the fermentative Lactobacillus flora. This effect is amplified still further by changes in the composition of the raw materials, by fluctuations in the ambient temperatures and by careless handling. The result of variations in the fermentation may adversely effect the flavour and appearance of the bread and the process overall is less convenient.

More usually, lactic acid bacteria fermentations are initiated by the addition of so called starter cultures. Starter cultures are concentrates of lactic acid bacteria which may be used as an addition to a food directly, or after subculturing and multiplication in the factory. Starter cultures are usually offered in either of two forms:

1) As a deep frozen concentrate containing $10^{10}$ cells per g or more. This requires distribution and storage at extremely low temperatures of below -30°C. A special type of freezer cabinet is required at the end user's premises. This limits application to scale large use as in modern dairies.

2) As a freeze dried powder. Freeze drying or lyophylisation is an expensive process and only applicable to a limited number of strains. Therefore freeze dried powders are only used to a limited extent.

Spray drying is unsatisfactory since at the high temperatures applied the bacteria are damaged or killed. In practice freeze drying is much too expensive for this purpose. The cost of drying is even more critical with lactic acid bacteria than with yeast since due to their physiological properties and growth requirements it will always be more expensive to grow the same amount of lactic acid bacteria biomass.

It has been proposed in EPO131114 to mix wet concentrate of lactic acid bacteria with a powdery carrier eg. skim milk powder of flour with post-drying to reduce the moisture levels sufficiently. This process is carried out in a fluidised bed. The cells are thus exposed to relatively high temperatures for some time. A lower temperature to minimise this damaging effect is uneconomical, since air of a very low dew point is required to generate sufficient drying activity.

According to the method described for the use of this previously proposed starter culture in sour-dough bread making, a batch of sour-dough sufficient for a week's supply must first be prepared before baking operations for the week can commence, a daily portion of the fermented sour-dough being taken from the week's previously prepared supply. This preliminary operation is slow, taking as long as 48 hours and requiring a special fermenter vessel big enough to make a weekly batch of sour-dough and capable of maintaining control of fermentation conditions. Variations can nevertheless take place in the bacterial flora as it develops during the week, with the possibility of adverse effects on the quality of the later prepared batches of bread.

Water activity is of importance for the growth behaviour of any microorganism. Cell activity is favoured by high water activities; Lower water activities will slow down metabolic activity. Extreme conditions will kill cells which however survive for very long time if they are really dry. Under these conditions the cells are dormant; no metabolism will occur.

During any drying process, cells are brought from a very wet state to a very dry one. The intermediate stages of water activity will have a negative effect on the cells. Hence it is important that the transition from very wet to dry is as quick as possible. Furthermore other unfavourable conditions, like temperature increases or low temperatures (as during spray drying) and ice crystals (freeze drying), should be avoided.

Notwithstanding the high viable count originally introduced into the slurry, the effect of drying in a warm atmosphere unavoidably reduces viable count and great care is required in packaging to avoid excessive loss of viability before use.

The present invention fulfils the need for an improved process for the preparation of dry ambient stable preparations of lactic acid bacteria capable of surviving storage conditions in small scale food companies, in addition to farms and large scale bakeries and in particular the production of stable, viable dried inoculum as easy to handle as modern dried baker's yeast in the sour dough method of bread making.

The present invention therefore provides a process for the preparation of a supported microflora composition of improved ambient stability, suitable for use as an inoculum, the constituents of which at least comprise an inert, subdivided support and an aqueous suspension of microflora, which composition has as a result of its watercontent and proportions of constituents a low wateractivity, while it contains an amount of pre-selected sugar, characterised by the following steps:

(1) preparing an aqueous dispersion of microflora with a concentration of 2-10 wt%

(2) adding at least one sugar, selected from the group consisting of sucrose, maltose and glucose to the dispersion of (1)

(3) drying a support material to a water content of less than 5 wt%

(4) mixing of the dried support of (3) with the aqueous dispersion of (2) providing a composition with a wateractivity of 0.3 or below and a sugar content of 0.1-10 wt%.

The biological benefit of predrying is that the cells which in most cases are of a mesophilic nature are not subjected to elevated temperatures in excess of about 30°C at any stage of the process and avoid heat damage, not necessarily reflected in cellular death with lethal effect but in sublethal effects which lower the functionality of the cells by enzyme inactivation and membrane permeability. Preferably four eg. rye flour, or other support is pre-dried to a water content below 5%, more preferably below 1%.

From DE 462,254 it is known, that by applying a desiccant moisture can be bound in supported microflora compositions resulting in dried compositions.

DE 481,614 discloses sour-dough cultures, wherein waterbinding compounds are used in order to provide dry compositions.

A similar process is disclosed in DE 481,615. However, according to this patent also sugars, such as dextrose, invert sugar or malt-extract can be incorporated in the mixture. In this way crust-formation is avoided. From S.E. Gilliland, Bacterial Starter Cultures for Foods, CRC-Press Inc. (1985) page 155 it is known that the addition of monosodium glutamate, lactose or sucrose might provide some protection to cultures of lactobacilli.

An advantage of the process according to the present invention is that, whereas supported microorganisms are extremely susceptible to reduction in viable count in the usual drying processes including fluidized drying processes even at moderately elevated temperatures, flour and many other powdered support materials may be much more strongly heated to provide a substrate, without deterioration of its composition or physical condition. The pre-drying step may also be more economical at high temperatures. Moreover it was found that the addition of the sugars mentioned in the step indicated had unexpected merits.

In practice, viable counts as high as $10^{10}$ per gramme may be obtained in products of the present invention. Concentrations of 2-10% in the slurry in conjunction with viable counts of $10^9$-$2 \times 10^{11}$/gm, particularly in the case of Lactobacilli strains, providing a dry concentration of 0.1 to 20% of microflora in the product, are preferred.

The supported Lactobacilli in the present invention include accelerants in the form of sucrose, maltose and/or glucose for promoting shelf-life and/or fermentation activity. However, also bread improvers having an enzyme basis, can be present. The sucrose maltose or glucose is present in an amount of 0.1 to 10% by weight of the product, or 1M to 2M concentration, and is incorporated beforehand in a ready mixed composition with the supported culture. The supported culture may also include yeasts and the present invention provides supported flora for use in sour dough preparation in which such accelerants and additives are incorporated.

The water activity of the supported flora products of the invention is 0.3 or less, particularly 0.2 or less. Improved storage life is provided surprisingly, with the presence of the sugars mentioned which on addition promotes cell stability during mixing and rehydration.

For storage purposes the products of the present invention are preferably sealed in tight packages, preferably in the absence of air, but good shelf-life may also be provided in air-permeable but moisture-proof packages. The packaging may be in measured amounts providing easy daily dispensing.

By this means substantially constant conditions of the fermentation are enjoyed from day to day, and for example the fermentation in sour-dough bread making can be completed within a normal day's work since a relatively small quantity only is needed and special devices for controlling the preparation of large weekly batches of sour-dough fermentation using supported Lactobacillus flora are no longer needed.

The present invention therefore also provides a method for making of sour-dough wherein batches of fermented dough are freshly prepared by inoculation with a supported Lactobacillus compositions made in accordance with the process of the invention. preferably made in accordance with the process of the invention.

Preferably the fermentation step is conducted as quickly as possible. To this end the amount of innoculant used in the preparation of each batch of sour-dough should preferably be substantially greater than hitherto recommended, to complete the fermentation in 24 hours. For example 1 to 20% by weight of dry matter of the inoculum is added to the flour used in preparation of the sour-dough starter material. The inoculum should have a viable count of at least $10^9$ per gramme of support, preferably above $10^{10}$ per gramme. The temperature of fermentation should preferably be maintained between 25 and 40°C, according to the temperature sensibility of the culture. The amount of water used in preparation of the sour-dough is preferably 1-2 parts of flour per part of water by weight. The fermentation is preferably completed in a single step in which the proportion of components is predetermined and incorporated in one operation, that is all the flour and all the water being added at a single stage with all the inoculum. By this means the sour-dough fermentation is fully controlled and the operation completed with a high inoculum level in a single step and with better temperature control and providing a short total fermentation time of from 3 to 18 hours.

This use of the products made in accordance with the invention therefore represents the adoption of daily fresh preparation from inoculum providing instantaneous growth and acid formation, in a similar manner to the application in bread making of baker's yeast fermentation and in fermentations applied in the dairy industry for example in the preparation of yoghurt.

The Lactobacillus strain used in the present invention may be selected on the basis of the desired flavour in the bread and/or its taste and keepability.

The method of sour dough preparation of the invention is suitable for use in conjunction with other supported lactobacillus compositions, or with frozen or freeze-dried starter cultures which have been made available for use in sour-dough bakery practice, although Lactobacilli are difficult to freeze dry on an industrial scale and frozen materials necessitate deep freezer facilities on the baker's premises.

The supported product of the present invention is also suitable for application in other food processes for example in the production of meat products with long shelf-life eg. Continental-type sausages such as cervelat, mortadella and salami, pickles and sauerkraut. For other applications of lactic acid bacteria to which the products of the present invention may be applied see Steinkraus, Handbook of Indigenous Fermented Foods. Further applications include silage manufacture in which lactic acid bacteria are used, suitable for application of other lactic acid bacteria eg. Pediococci, Streptococci, Leuconostocs and Bifidobacteria. Mixed strains may be used, particularly of yeast species and Lactobacilli.

Example 1

A stock of Lactobacillus species (NRRL B-18,368 May 11, 1988) was inoculated into 500 ml of De Man, Rogosa and Sharpe Broth. This was incubated overnight at 30°C to a cell density of about $2 \times 10^9$ per ml and the resulting volume used in turn to inoculate a stirred fermenter containing Lactobacillus medium with a composition given in Table 1, at a temperature of 32°C with incubation under nitrogen and a pH 5.8 with

$u_{max}$ = 0.7 h[1], yielding 8g/l biomass with a viable count range of $5 \times 10^9$ to $2 \times 10^{10}$/ml.

The resulting culture broth was centrifuged, giving approximately a 20 fold increase in cell density up to $2 \times 10^{11}$/g viable counts. When cooled to 0 to 5°C the concentrate could be stored for several days without deterioration.

Sugar was added to the concentrate giving a final molarity of approximately 2 and the composition intermittently mixed with rye flour previously dried to less than 1% moisture in a spiral dryer and cooler unit ex Werner Pfleiderer, West Germany, giving an $a_w$ value for the product of 0.2. The product was then packed in air tight plastic bags and was found to remain satisfactory active for several months.

## TABLE 1

### COMPOSITION OF LACTOBACILLUS MEDIUM

| | g |
|---|---|
| Glucose anhydrous | 60 |
| yeast extract | 15 |
| beef extract | 30 |
| diammonium citrate | 6 |
| $MgSO_4.7_2O$ | 0.3 |
| $MnSO_4.H_2O$ | 0.2 |
| $Na_2HPO_4.2H_2O$ | 6.0 |
| tapwater to 1000 ml | |

sterilization conditions 20 minutes at 120°C

Resulting pH = 6.3

Example 2

A sour-dough batch was prepared from 500 g of rye flour having an ash content 1.1%, 200 g of dry powdered Lactobacilli starter prepared as described in Example 1 and having a viable count of $6 \times 10^9$ per ml and 560 g of water. The resulting dough was fermented for 4 hours at 30-32°C in proofing cabinet, when the initial pH decreased from approximately 6 to approximately 4 and the Total Titratable Acidity increased from approximately 2.5 to approximately 10. pH was measured by dispersing 10 g of dough in 100 mls of water and Total Titratable Acidity by the amount of 0.1N NaoH required to titrate 10 g of dough dispersed in 100 mls of water to pH 8.5.

Bread dough was mixed from the following ingredients;

1260 g sour dough prepared as above
700 g rye flour
600 g wheat flour
30 g pressed yeast
40 g salt
780 g water

The dough was maintained at 27 to 28°C and successively kneaded for 8 to 14 minutes according to the mixture used, and rested in a first proof for half an hour. After moulding, shaping and rounding into loaves where it was given a final proof at 30°C and 70% relatively humidity of approximately an hour, the loaves were baked for 60 minutes at a temperature progressively decreased from 260°C to 220°C during baking.

5

The resulting bread had a good crumb texture and excellent sourish taste, with a pH = 4.4 and TTA = 7.5.

Satisfactory loaves were also baked using up to half the total rye flour in the sour dough, according to the acidity required in the product.

Example 3

The effect of sucrose on the survival on storage of supported cells prepared in accordance with the invention, was observed at various levels of moisture in the product and sucrose in the suspension. The supported product was prepared as described in Example 1. Rye flour was pre-dried to 1% moisture as the support medium, onto which was sprayed two percent of an aqueous suspension of the same Lactobacillus strain as in Example 1, sucrose being included in the suspension at various molarities for different concentrations of the cell suspension. A control series with no added sucrose was included in the tests. The moisture content of the products obtained ranged from approximately 2% for the lower sucrose and cell concentrations, equivalent to a water activity of approximately 0.1, and progressively increased to 7% moisture, equivalent to $A_w = 0.39$.

The viable count varied little in the suspensions, between the control sample at 2.3-3.0 x $10^{11}$, to a range for the test samples from approximately 1.3 to 3.0 x $10^{11}$. The count for the supported samples ranged from 4.5 to 13x$10^9$ for the control samples and was in the range 2.5-28 x $10^9$ for the sugar-containing samples, a progressive increase in these later series being observed with increasing cell concentration.

The fresh counts were compared with repeated counts on the products after nine weeks storage at 35°C and 12 and 26 weeks storage at 25°C, in air and nitrogen. The comparison was expressed as percentage survival in each case. Survival of the order of 50% or more is regarded as satisfactory. Results appear in the accompanying Table 2.

6

EP 0 298 605 B1

**TABLE 2**

| Molarity of Sucrose in suspension | Suspension with Sucrose added w/w | Survival percentage of cells in product after storage (1%) | | | | | |
|---|---|---|---|---|---|---|---|
| | After correction for sucrose | After 9w, 35C | | After 12w, 25C | | After 26w, 25C | |
| | | Air | $N_2$ | Air | $N_2$ | Air | N2 |
| 0 | 2 | 19.2 | – | 2.1 | – | 1.0 | 38.1 |
| | 5 | * | 6.3 | 12.5 | – | 0.94 | 16.9 |
| | 7.5 | * | * | 2.8 | – | * | 6.0 |
| 1.0 | 1.5 | 58.3 | 66.7 | 58.3 | – | 17.5 | 69.4 |
| | 3.8 | 22.7 | 40 | 31.8 | – | 7.9 | 84.5 |
| | 5.7 | 5 | 30.8 | 33.8 | – | 6.5 | 84.0 |
| | 7.6 | 5.9 | 15.9 | 28.2 | – | 13.5 | 52.9 |
| | 9.5 | * | 1.1 | 10 | – | 0.89 | 7.9 |
| 1.5 | 1.3 | 64 | 76 | 44 | – | 92 | N.D. |
| | 3.2 | 73.2 | 55.4 | 58.9 | – | 78.6 | N.D. |
| | 4.8 | 53 | 31 | 42 | – | 24 | 78 |

TABLE 2

Survival percentage of cells in product after storage (1%)

| Molarity of Sucrose in suspension | Suspension with Sucrose added w/w After correction for sucrose | After 9w, 35C Air | After 9w, 35C N2 | After 12w, 25C Air | After 12w, 25C N2 | After 26w, 25C Air | After 26w, 25C N2 |
|---|---|---|---|---|---|---|---|
| 1.5 (cont.) | 6.4 | 41.8 | 87.3 | 37.3 | – | 51 | 61.8 |
|  | 7.9 | 25.9 | 41.5 | 34.1 | – | 70.6 | 88.2 |
|  | 9.5 | 13.2 | 43.2 | 25.9 | – | 24.5 | 59.1 |
| 2.0 | 1.0 | 58.1 | 90.3 | 26.1 | – | 64.5 | 64.5 |
|  | 2.6 | 64.4 | 72.9 | 50.8 | – | N.D. | N.D. |
|  | 3.8 | 69.3 | 79 | 62.9 | – | N.D. | N.D. |
|  | 5.2 | 68.9 | 68.9 | 52.7 | – | 87.8 | N.D. |
|  | 6.5 | 34 | 52 | 28 | – | 86.7 | 61.3 |
|  | 7.8 | 18.7 | 55.3 | 37.3 | – | 34 | N.D. |
|  | 10.4 | 26.7 | 47.9 | 40.7 | – | 62.8 | N.D. |

N.D. - No decrease

* - below 0.1

From the results the following points can be made:

For good storage stability at 25°C in the presence of air 1 molar of sucrose is clearly insufficient. 1.5 M of sucrose gave good storage stability at dosing levels of the cell-sucrose mixture up to 12.5%, which is 6.5% of the original cell suspension resulting in a preparation containing $10^{10}$ cells/g.

With increasing sucrose molarity the differences in survival rates between samples stored under $N_2$ and in air decreased and vanished in the presence of 2 M of sucrose.

Even at the same $A_w$ value, survival percentages increased with increasing sucrose molarity. The role of sucrose is thus more than only that of $A_w$ lowering agent.

## Example 4

Example 2 was repeated to test the influence of different methods of introducing sucrose into the mix. This was carried out with the compositions with 2 M sucrose and dosage levels of 7.5 and 20%.

The methods tested were :

A. 2 Molar of sucrose (final concentration in cell suspension) was added to the cell suspension, then the mixed suspension was sprayed onto the rye flour carrier.

B. Cell suspension was sprayed onto rye flour then dry sucrose was mixed with the flour in an amount equalling on a weight basis the amount of sucrose added in A.

C. An amount of sucrose equalling on a weight basis the amount of sucrose added in A was mixed with the rye flour after which the cell suspension was sprayed onto the flour/sucrose mixture.

Results showed that the best survival in products prepared with the same amount of sucrose was obtained with Method A. The survival percentages obtained with the other two methods were higher than the survival percentages in products prepared without sucrose.

## Example 5

Using cultivated and concentrated Lactobacillus strain (as in Example 1) the following compounds were tested: Glucose, Lactose, Maltose, Sucrose, Mannitol, Sorbitol, GLycerol, Diethyleneglycol (DEG) and Monosodiumglutamate (MSG). To the concentrated cells appropriate amounts were added to obtain the required molarity strengths. The molarity strengths tested were 1 M and 2 M with all components except for Mannitol and Lactose which were tested on 1 M strength only because of their poor solubility. The amounts of cell + compound mixtures was dosed in such a way that in all cases 2% of the original cell concentrate was mixed with the pre-dried rye flour. In all samples moisture content and $A_w$ was measured. Samples were stored at 25°C and 35°C in the presence of air and viable counts determined after 12 and 26 weeks with 25°C and after 4 and 9 weeks with 35°C.

Results are given in Table 3, with V.C.'s determined after production given as such and V.C.'s determined during storage expressed as survival percentages in which V.C. after production is stated 100%. In the presence of 2 M good protection (> 50%) was found with Glucose, Maltose and Sucrose, slight protection was found with Sorbitol and MSG (12.2 and 18.9) whereas Glycerol and DEG showed no protective effect at all. In the presence of 1M, Mannitol and Lactose showed some protective effect.

## TABLE 3

Survival percentages of L.brevis var Lindneri during storage
in the presence of air

| | Viable Count after prod. | % survival, $N_{t-o}$= 100% | | | |
|---|---|---|---|---|---|
| | | 4 wks 35°C | 9 wks 35°C | 12 wks 25°C | 26 wks 25°C |
| Glucose 1.2 M | $2.2 \times 10^9$ | 127.3 | 45.4 | 86.4 | 36.4 |
| 2.4 M | $2.1 \times 10^9$ | 152.4 | 57.1 | 71. | 76.2 |
| Lactose 1.1 M | $2.6 \times 10^9$ | 119.2 | 28. | 69.2 | 9.6 |
| Maltose 1.2 M | $2.4 \times 10^9$ | 113.3 | 28.3 | 43.3 | 27.0 |
| 2.1 M | $3.0 \times 10^9$ | 112.5 | 39.6 | 91.7 | 62.5 |
| Sucrose 1.1 M | $3.3 \times 10^9$ | 113.8 | 46.1 | 51.5 | 43.1 |
| 2.4 M | $3.2 \times 10^9$ | 116.1 | 64.5 | 64.5 | 63.1 |
| Mannitol 1.2M | $2.0 \times 10^9$ | 47.6 | 13.5 | 32.1 | 6.5 |
| Sorbitol 1.2M | $2.0 \times 10^9$ | 65.0 | 19.5 | 55.0 | 15.5 |
| 2.4M | $2.7 \times 10^9$ | 18.5 | 8.9 | 31.5 | 18.9 |
| Glycerol 1.2M | $2.4 \times 10^9$ | 34.2 | 4.1 | 26.7 | 3.7 |
| 2.4M | $1.6 \times 10^9$ | 0.4 | 9.4E-3 | 9.4E-1 | 1.2E-7 |

## TABLE 3

### Survival percentages of L.brevis var Lindneri during storage in the presence of air

| | | Viable Count after prod. | 4 wks 35°C | 9 wks 35°C | % survival, $N_{t-o}$ = 100% 12 wks 25°C | 26 wks 25°C |
|---|---|---|---|---|---|---|
| DEG | 1.1 M | $2.2 \times 10^9$ | 32.7 | 1.4 | 10.5 | 3.4E-2 |
| | 2.3 M | $1.7 \times 10^9$ | 5.1 | 2.1E-1 | 2.0 | 1.3E-1 |
| MSG | 1.2 M | $2.7 \times 10^9$ | 70.4 | 22.2 | 48.1 | 7.4 |
| | 2.4 M | $2.7 \times 10^9$ | 96.3 | 36.7 | 92.6 | 12.2 |
| Water | | $2.3 \times 10^9$ | 47.8 | 3.8 | 21.7 | 4.0E-1 |

In all samples $A_w$ = 0.1

Example 6

Preparation of cell concentrate: 2500 ml sterile MRS-broth was inoculated with 2.5 ml of an overnight culture of L. acidophylus in MRS at 35°C and incubated overnight at 35°C. The culture was concentrated

11

EP 0 298 605 B1

by centrifugation for 10 min. at 7000 rpm and the cells suspended in part of the supernatant to obtain 115 gr of cell concentrate. The waterphase of the concentrate was 90%.

Prearation of product. Using a Lödige mixer 3 batches of product according to the invention were prepared as follows:

1. 30 gr of cell concentrate was mixed with 35.8 gr sucrose (ca 2M sucrose) and after 1 hr of storage at 4°C mixed with 1.5 Kg of pre-dried milkpowder.

2. 30 gr of cell concentrate was mixed with 1.5 Kg pre-dried milkpowder and subsequently mixed with 35.8g sucrose.

3. 1.5 Kg of pre-dried milkpowder was mixed with 35.8g sucrose, then 30 gr of cell concentrate was mixed.

Storage tests: With the samples storage tests were carried out at 25°C and 35°C both in the presence of air and under nitrogen. Viable counts (V.C.'s) were determined after production and after 4, 12 and 26 wks storage at 25°C and after 4 and 9 wks storage at 35°C.

Results (see Table 4). L. acidophylus survived best in sample 1 during storage at 25°C and 35°C both in the presence of air and under $N_2$ (>50% of survivors). In the samples 2 and 3 survival was better during storage under $N_2$ than during storage in the presence air.

## Table 4

| | 4 wks 25°C | 12 wks 25°C | 26 wks 25°C | 4 wks 35°C | 9 wks 35°C |
|---|---|---|---|---|---|
| **Air** | | | | | |
| Sample 1 | $1.5 \times 10^8$ | $1.2 \times 10^8$ | $1.4 \times 10^8$ | $1.2 \times 10^8$ | $9.9 \times 10^7$ |
| 2 | $1.1 \times 10^8$ | $8.7 \times 10^7$ | $2.5 \times 10^7$ | $7.9 \times 10^7$ | $10^7$ |
| 3 | $1.2 \times 10^8$ | $7.6 \times 10^7$ | $1.0 \times 10^7$ | $8.7 \times 10^7$ | $1.7 \times 10^7$ |
| **$N_2$** | | | | | |
| Sample 1 | $1.5 \times 10^8$ | $1.5 \times 10^8$ | $1.2 \times 10^8$ | $1.5 \times 10^8$ | $1.1 \times 10^8$ |
| 2 | $1.3 \times 10^8$ | $1.3 \times 10^8$ | $9.5 \times 10^7$ | $1.4 \times 10^8$ | $6.3 \times 10^7$ |
| 3 | $1.8 \times 10^8$ | $1.4 \times 10^8$ | $5.6 \times 10^7$ | $1.2 \times 10^8$ | $8.4 \times 10^7$ |

**V.C. immediately after production:   $A_w$**

| | | |
|---|---|---|
| Sample 1 | $1.7 \times 10^8$ | 0.13 |
| 2 | $1.7 \times 10^8$ | |
| 3 | $1.7 \times 10^8$ | |

Example 7

Microorganisms:

1. Saccharomyces cerevisiae, Böcker starter isolate
2. Candida kruzoi, sour dough isolate
3. S. cerevisiae, RZ-starter - DHW isolate
4. S. cerevisiae, baker's yeast.

12

Preparation of cell concentrate. With each strain 1 litre of sterile yeast extract-glucose-maltextract-broth was inoculated with $10^5$ cells/ml. The broth was distributed over three 1 litre erlenmeyer flasks and incubated at 25°C under shaking conditions. After 48 hours the cultures were concentrated by centrifugation for 10 min. at 6000 rpm and the cells suspended in part of the supernatant to obtain 80 gr of cell concentrates. The waterphases in the concentrates were 83, 78, 84 and 90% respectively.

Preparation of product.

With the cell concentrates products were prepared by mixing 30 g cell concentrate with 1.5 Kg of pre-dried rye flour, both without sugar and in the presence of 2M sugar viz.

Sample 1: strain no. 1: 30 gr concentrate

Sample 2: strain no. 1: 30 gr concentrate + 33 gr sucrose

Sample 3: strain no. 2: 30 gr concentrate

Sample 4: strain no. 2: 30 gr concentrate + 31 gr sucrose

Sample 5: strain no. 3: 30 gr concentrate

Sample 6: strain no. 3: 30 gr concentrate + 28 gr sucrose

Sample 7: strain no. 4: 30 gr concentrate

Sample 8: strain no. 4: 30 gr concentrate + 35.8 gr sucrose

Storage tests: Storage tests were carried out at 25°C and 35°C both in the presence of air and under nitrogen. Viable counts were determined after production and after 12 and 26 wks of storage at 25°C and after 4 and 9 wks at 35°C.

Results (see Table 5): Results are given as V.C.'s determined after production as such and V.C.'s determined during storage expressed as survival percentages in which V.C. after production is stated as 100%.

Conclusion: From these results it is clear that sucrose has a positive effect on the survival of yeasts in the process according to the invention and during storage at ambient temperature in the presence of air.

TABLE 5

<u>SURVIVAL OF YEASTS IN THE CDC PROCESS</u>

| Gas | | | | VC after production | 12 wks 25°C | 4 wks 35°C | 9 wks 35°C |
|---|---|---|---|---|---|---|---|
| Air | <u>Sour dough yeasts</u> | | | | | | |
| | Böcker isolate | 0 M sucrose | | $5.0 \times 10^6$ | 2.6 E-1 | 4.2 E-1 | 2.8 E-4 |
| | | 2 M | " | $4.5 \times 10^7$ | 88.9 | 71.1 | 93.3 |
| | C. kruzei (SD) | 0 M | " | $2.8 \times 10^7$ | 1.4 | 2.7 | 1.1 E-2 |
| | | 2 M | " | $9.8 \times 10^7$ | 94.9 | 90.8 | 142 |
| | S. cerevisiae | | | | | | |
| | (DHW) | 0 M | " | $7.3 \times 10^6$ | 8.6 E-2 | 6.8 E-2 | 1.9 E-3 |
| | | 2 M | " | $4.7 \times 10^7$ | 72.3 | 51.1 | 59.6 |
| | <u>Bread yeast</u> | | | | | | |
| | S. cerevisise | 0 M sucrose | | $3.0 \times 10^5$ | <3.3 E-2 | 8.3 E-1 | 3.3 E-2 |
| | | 2 M | " | $8.7 \times 10^6$ | 35.6 | 17.2 | 3.0 |

EP 0 298 605 B1

$N_2$ **Sour dough yeasts**

| | | | | | |
|---|---|---|---|---|---|
| Böcker isolate | 0 M sucrose | - | 34.0 | 46.0 | 32.0 |
| | 2 M " | - | 97.8 | 68.9 | 86.7 |
| C. kruzei (SD) | 0 M " | - | 64.3 | 89.3 | 50.0 |
| | 2 M " | - | 102 | 99.0 | 112 |
| S. cerevisiae (DHW) | 0 M " | - | 21.9 | 63.0 | 27.4 |
| | 2 M " | - | 63.8 | 97.9 | 66.0 |

**Bread yeast**

| | | | | | |
|---|---|---|---|---|---|
| S. cerevisiae | 0 M sucrose | - | 6.3 | 3.3 | 1.1 E-1 |
| | 2 M " | - | 36.8 | 60.9 | 32.2 |

EP 0 298 605 B1

METHODS AND MEDIA

Determination of waterphase percentage in cell concentrate

In triplicate weigh in glass jars 10 g of the cell concentrate and heat in the microwave oven under shaking in such a way that all the moisture has been evaporated without burning the cells.

Determine the amount of waterphase and calculate the average of the three analyses.

Calculation of the amount of $A_w$ lowering compound for the adjustment to a certain molarity in a cell concentrate

Determine the % of waterphase of the cell concentrate according to the above method, state W. To adjust 100 g of this concentrate to a certain molarity (a) the required amount of solute (S) can be calculated from the formula:

$$S = \frac{W}{100 \times S.W. - a \times M.W.} \times a \times M.W.$$

in which
W = % of waterphase in the cell concentrate
S.W = specific weight
a = required molarity of solute
M.W. = molecular weight

Determination of total titratable acids (TTA)

Definition: The TTA of a product is the number of ml of a 0.1 M NaOH solution necessary to neutralize to pH 8.5 the amount of acids present in 10 g of the product.

Method: Weigh about 10 g of dough and add an amount of distilled water to obtain a $10^{-1}$ solution. Homogenize for 45 seconds and take 50 g of the suspension and add another 50 g of distilled water. Titrate the suspension with 0.1 N NaOH until pH 8.5. Mls. 0.1 N NaOH x 2 is the TTA of the product.

**Claims**

**1.** Method for preparing a supported microflora composition of improved ambient stability, suitable for use as an inoculum, the constituents of which at least comprise an inert, sub-divided support and an aqueous suspension of microflora, which composition has as a result of its watercontent and proportions of constituents a low wateractivity, while it contains an amount of pre-selected sugar, characterised by the following steps:
(1) preparing an aqueous dispersion of microflora with a concentration of 2-10 wt%
(2) adding at least one sugar, selected from the group consisting of sucrose, maltose and glucose to the dispersion of (1)
(3) drying a support material to a water content of less than 5 wt%
(4) mixing of the dried support of (3) with the aqueous dispersion of (2) providing a composition with a wateractivity of 0.3 or below and a sugar content of 0.1-10 wt%

**2.** Method according to claim 1, wherein the water content of the support in step (3) is adjusted to less than 1 wt%.

**3.** Method according to claim 1, wherein the microflora comprises mesopholic bacteria cells.

**4.** Method according to claims 1-3, wherein the viable count of the suspension ranges from $10^9$-$2 \times 10^{11}$.

**5.** Method according to claim 1, wherein the microflora comprises lactobacillus species.

EP 0 298 605 B1

6. Method according to claim 1, wherein the microflora comprises yeast species.

7. Method according to claim 1, wherein the microflora comprises a mixture of yeast and lactobacilli.

8. Method according to claims 5-7, wherein the viable count of the suspension ranges from $10^9$-$10^{12}$.

9. Method for making a sour dough for bread making, wherein batches of fermented dough are freshly prepared by inoculation with supported lactobacillus compositions, obtained as result of the processes according to claims 5-7.

**Patentansprüche**

1. Verfahren zur Herstellung einer Mikroflora-Trägerzusanmensetzung verbesserter Umgebungsstabilität, die zur Verwendung als Impfkultur geeignet ist, deren Bestandteile mindestens einen inerten, unterteilten Träger und eine wäßrige Suspension der Mikroflora umfassen, wobei die Zusammensetzung aufgrund ihres Wassergehaltes und des Verhältnisses der Bestandteile eine geringe Wasseraktivität hat, während sie eine Menge eines ausgewählten Zuckers enthält, gekennzeichnet durch die folgenden Schritte;
   (1) Herstellen einer wäßrigen Dispersion der Mikroflora mit einer Konzentration von 2 bis 10 Gew.-%,
   (2) Zugeben mindestens eines Zuckers, ausgewählt aus der aus Saccharose, Maltose und Glucose bestehenden Gruppe, zur Dispersion von (1),
   (3) Trocknen eines Trägermaterials auf einen Wassergehalt von weniger als 5 Gew.-%,
   (4) Mischen des getrockneten Trägers von (3) mit der wäßrigen Dispersion von (2) unter Bildung einer Zusammensetzung einer Wasseraktivität von 0,3 oder weniger und eines Zuckergehaltes von 0,1 bis 10 Gew.-%.

2. Verfahren nach Anspruch 1, worin der Wassergehalt des Trägers in Schritt (3) auf weniger als 1 Gew.-% eingestellt wird.

3. Verfahren nach Anspruch 1, worin die Mikroflora mesophile Bakterienzellen umfaßt.

4. Verfahren nach den Ansprüchen 1 bis 3, worin die lebensfähige Zählung der Suspension zwischen $10^9$ bis $2 \times 10^{11}$ beträgt.

5. Verfahren nach Anspruch 1, worin die Mikroflora Lactobacillus-Spezies umfaßt.

6. Verfahren nach Anspruch 1, worin die Mikroflora Hefe-Spezies umfaßt.

7. Verfahren nach Anspruch 1, worin die Mikroflora eine Mischung aus Hefe und Lactobacilli umfaßt.

8. Verfahren nach den Ansprüchen 5 bis 7, worin die lebensfähige Zählung der Suspension zwischen $10^9$ bis $10^{12}$ beträgt.

9. Verfahren zur Herstellung eines Sauerteiges zur Brotbereitung, worin Chargen von fermentiertem Teig durch Animpfen mit Lactobacillus-Trägerzusammensetzungen, die nach den Verfahren gemäß den Ansprüchen 5 bis 7 erhalten wurden, frisch hergestellt werden.

**Revendications**

1. Procédé pour la préparation d'une composition de microflore sur support de réaction, ayant une meilleure stabilité dans des conditions ambiantes, appropriée pour être utilisée en tant qu'inoculum, dont les constituants comprennent au moins un support de réaction inerte subdivisé et une suspension aqueuse de microflore, ladite composition ayant une faible activité aqueuse en raison de sa teneur en eau et des proportions des constituants, tout en contenant une quantité de sucre présélectionné, caractérisé par les étapes suivantes :
   (1) préparer une dispersion aqueuse de microflore, avec une concentration de 2 - 10 % en masse;
   (2) ajouter au moins un sucre, sélectionné à partir du groupe composé du saccharose, du maltose et du glucose à la dispersion obtenue au point (1);

17

(3) sécher le support de réaction jusqu'à ce que sa teneur en eau soit inférieure à 5 %;

(4) mélanger le support de réaction obtenu au point (3) avec la dispersion aqueuse obtenue au point (2), ce qui fournit une composition avec une activité aqueuse de 0,3 ou inférieure et une teneur en sucre de 0,1 à 10 %.

2. Le procédé selon la revendication 1, dans lequel la teneur en eau du support de réaction obtenu au point (3) est ajustée à un niveau inférieur à 1% en masse.

3. Le procédé selon la revendication 1, dans lequel la microflore comprend des cellules de bactéries mésophiles.

4. Le procédé selon les revendications 1 à 3, dans lequel le nombre de cellules vivantes dans la suspension est compris dans la gamme allant de $10^9$ à $2 \times 10^{11}$.

5. Le procédé selon la revendication 1, dans lequel la microflore comprend des espèces de Lactobacille.

6. Le procédé selon la revendication 1, dans lequel la microflore comprend des espèces de levure.

7. Le procédé selon la revendication 1, dans lequel la microflore comprend un mélange de levure et de Lactobacilles.

8. Le procédé selon les revendication 5 à 7, dans lequel le nombre de cellules vivantes dans la suspension est compris dans la gamme allant de $10^9$ à $10^{12}$.

9. Procédé de fabrication d'une pâte aigrie pour la panification, dans lequel des lots de pâte à pain fermentée sont fraîchement préparés par inoculation avec des compositions de Lactobacille sur support de matériau, obtenues grâce aux procédés selon les revendications 5 à 7.